# EUROPEAN PATENT APPLICATION

(11) **EP 1 321 768 A1**
(43) Date of publication of application: **25.06.2003**
(21) Application number: 01129389.1
(22) Date of filing: 18.12.2001
(51) Int. Cl.: G01N 33/50, G01N 33/74, G01N 33/68

(54) **Diagnosis of endometriosis from menstrual blood**

(71) Applicant: Roche Diagnostics GmbH, 68305 Mannheim (DE); F.HOFFMANN-LA ROCHE AG, 4070 Basel (CH)
(72) Inventor: Leyendecker, Gerhard, 64283 Darmstadt (DE)

(57) **Abstract**

The present invention relates to a method for diagnosing endometriosis. The method comprises obtaining a sample of menstrual blood, measuring basal cells in said sample or measuring at least one gene product typical for cells of the basal endometrium in said sample and correlating said measurement to the diagnosis of endometriosis.

## Description

The present invention relates to a method for diagnosing endometriosis. The method comprises obtaining a sample of menstrual blood, measuring basal cells in said sample or measuring at least one gene product typical for cells of the basal endometrium in said sample and correlating said measurement to the diagnosis of endometriosis.

### Background of the invention

Endometriosis is a common and painful disorder characterized by the growth of endometrial cells at extra uterine (ectopic) sites. Ectopic implantation and growth of endometrical tissue is found on the peritoneum of the abdominal wall and at the outer surface of various organs, including primarily, the lower bowel, ovaries and fallopian tubes (Vigano, P., et al., Fertil Steril 56 (1991) 894-9).

Various theories on origin and etiology of endometriosis have been put forward and are currently discussed in the corresponding scientific literature.

In a first hypothesis, endometriosis is considered an autoimmune disease. This theory is based on the findings that in many sera of patients antibodies against "self antigens" are found. Several diagnostic efforts are based on the detection and measurement of auto-antibodies found in sera derived from endometriotic patients (WO 01/79846).

A second hypothesis on the etiology of endometriosis is the so-called "theory of ectopic implantation" Sampson, J. A., Am. J. Obstet. Gynaecol. 14 (1927) 422-429 whereas a third hypothesis explains endometriosis by "metaplasia" of endometric tissue (Meyer, R., Zbl. Gynäk. 43 (1919) 745-750). Several reports suggest that retrograde menstruation which may be linked to abnormal immune function, may play a role in establishing ectopic endometric lesions (Ishimura, T. and Masuzaki, H., Am J Obstet Gynecol 165 (1991) 210-4).

It has been recently found, that endometriosis and adenomyosis are variants of the same disease process with uterine peristalsis and hyperperistalsis being important causal factors. It was proposed that adenomyosis would result from the infiltration of basal endometrium into myometrial dehiscencies that were caused by chronic uterine peristalsis and hyperperistalsis and that the muscular component of adenomyosis would result secondarily from metaplasia of the infiltrating endometrial stroma (Kunz, G., et al., Hum Reprod 15 (2000) 76-82). It was furthermore suggested that endometriosis would result from the detachment of endometrial cells with a higher potential for proliferation and infiltrative growth and from their enhanced transtubal transport by uterine hyperperistalsis into the peritoneal cavity, where they implant on peritoneal surfaces (Leyendecker, G., et al., Hum Reprod 11 (1996) 1542-51; Leyendecker, G., et al., Hum Reprod Update 4 (1998) 752-62; Leyendecker, G., Hum Reprod 15 (2000) 4-7).

Endometriotic tissue is supported by and connected to the bodies blood system. In theory, independent of its "true" etiology, endometriosis might be diagnosed from blood by detecting therein marker-molecules which are indicative for this disease entity. Several approaches are known from the patent literature which focus diagnostic procedures for diagnosis of endometriosis based on the detection of marker molecules from blood or from tissue samples. E.g., WO 99/63116 describes the up-regulation of prothymosin in endometric tissue and describes approaches for use of prothymosin in the diagnosis of endometriosis, especially from blood or from samples of endometriotic biopsies.

However, it appears that to date for the field of endometriosis no biochemical or immunological marker is generally accepted or broadly used in clinical diagnostic routine despite the urgent need for such a tool.

Whereas in the attempts known from the art blood or blood constituents are primarily used in the diagnosis of endometriosis, it now has surprisingly been found that endometriosis can be diagnosed by analyzing menstrual blood.

### Summary of the invention

While there is a tremendous load of endometrial tissue which is shed off as a normal result of the menstrual cycle, it has now been established, that it is possible to detect gene products derived from a special layer of the endometrium, the so-called "zone IV basal endometrium", of which fragments are not or only rarely found in menstrual blood of women without endometriotic lesions but are frequently found in a menstrual blood sample of women suffering from endometriosis.

In a first embodiment the present invention therefore relates to a method for diagnosing endometriosis, said method comprising obtaining a sample of menstrual blood measuring cells of the basal endometrium in said sample or measuring at least one gene product typical for cells of the basal endometrium in said sample and correlating said measurement to the diagnosis of endometriosis.

Preferably, the gene product which is detected from such a basal cell is an mRNA or a polypeptide. Such polypeptide for example is easily detected by immunohistology or by aid of an immunoassay.

In a further preferred embodiment the present invention relates to a method for diagnosing endometriosis, said method comprising obtaining a sample of menstrual blood measuring the amount of at least one gene product typical for cells of basal endometrium in said sample, measuring the amount of a gene product of cells of the functionalis layers of the endometrium in said sample, assessing the relative amount of the gene product typical for basal endometrium to the gene product of the functionalis layers of the endometrium and correlating said relative amount to endometriosis.

### Detailed description of the invention

In order to fully understand and appreciate the procedure of and the progress achieved by the present invention, it is necessary to describe in some detail the various tissues comprised within the female uterus, their phylogenetic origin as well as their properties and changes during the course of the menstrual cycle.

The uterus is composed of two different organs, the inner archimetra and the outer neometra (Noe, M., et al., Hum Reprod 14 (1999) 190-7, especially Fig. 5 therein). The endometric epithelial cells, the endometrial stroma as well as the subendometrial myometrium (archimyometrium or functionals zone myometrium) are phylogenetically rather old tissues and therefore termed archimetrium. The later phylogenetic acquisition of muscular layers in addition to the myometrium, which are represented by the stratum vasculare and the stratum supravasculare, appears to be related to the forces that are required for parturition under the condition of viviparity.

Phylogenetically and ontogenetically, the archimetra or endometrial-subendometrial unit constitutes the oldest part of the uterus (hence its denomination archimetra) and is composed of the epithelial and stromal endometrium and the underlying stratum subvasculare of the myometrium with a predominantly circular arrangement of muscular fibers. While both, the endometrium and the subendometrial myometrium display a cyclic pattern of steroid hormone receptor expression, the two other layers of the myometrium, the outer stratum supravasculare with a predominantly longitudinal arrangement of muscular fibers and the stratum vasculare consisting of a three-dimensional mesh of short muscular bundles (Werth, R. and Grusdew, W., Arch. Gynäkol. 55 (1898) 325-409; Wetzstein, R., Arch. Gynecol. 202 (1965) 1-13), rather show a continuously high expression of both the estrogen receptor (ER) and the progesterone receptor (PR) throughout the cycle (Noe et al., supra). Only the archimetra is of paramesonephric origin, while the outer layers, the neometra, are of non-Müllerian origin (Werth and Grusdew, supra). There is an intimate structural contact between the stratum subvasculare and the stratum vasculare in that the muscular fibers of the two layers blend (Werth and Grusdew, supra). This transitional zone (TZ), which constitutes roughly the inner quart of the stratum vasculare, exhibits an intermediate cyclic pattern of steroid receptor expression (Noe et al., supra).

The functionalis is the endometrial tissue undergoing most changes during a menstrual cycle: In the first half of the menstrual cycle the functionalis is proliferating under the influence of estradiol and is, following the rise of progesterone after ovulation, transformed into the secretory endometrium and shed at the end of a non-pregnancy cycle. The functionalis is comprised of two layers (zone I and II functionalis). Layer III is an intermediate layer between functionalis and basalis. The basal zone IV endometrium is the deepest endometrial layer at the bottom of the glands. It does not undergo secretory transformation, it is usually not shed during menstruation, it is most active at the end of the cycle and has the potential of rebuilding the endometrium. All four layers are composed of epithelium and stroma (Padykula, H. A., et al., Biol Reprod 40 (1989) 681-90; Okulicz, W. C., et al., Biol Reprod 49 (1993) 24-32).

Layer IV of the endometrium is also called "basal endometrium". In the sense of the present invention "basal cells" or "basalis cells" or "cells of the basal endometrium" refer to cells of layer IV of the endometrium.

According to the present invention "functionalis" of "functionalis cells" are the cells comprised in layers I and II of the endometrium.

At the end of the secretory phase and at the time of the onset of menstruation mitotic and hence proliferative activity is only present in the basal zone IV of the primate endometrium, while in all of the functionalis and in the basal zone III mitotic quiescence prevails (Padykula, et al., supra; Okulicz, et al., supra). Furthermore, peristromal smooth muscle cells have been shown to be present in all superficial peritoneal endometriotic lesions and that they may result from stromal metaplasia of the shed and implanted endometrium (Anaf, V., et al., Hum Reprod 15 (2000) 767-71) indicating that, with respect to the main morphological components, i.e. endometrial epithelium, endometrial stroma and smooth muscular tissue, no principal difference exists between endometriosis and adenomyosis.

Surprisingly it has been found, that adenomyosis and endometriosis, result from the dislocation of basal endometrium - adenomyosis by direct infiltration of basalis into the myometrial wall and endometriosis following the detachment of fragments of zone IV basalis during menstruation and their transplantation into the peritoneal cavity. Even more surprisingly, it could be shown that diagnosis of endometriosis from menstrual blood is possible based on the specific detection of basal cells or of at least one gene product, which is typical for basal cells.

In a first embodiment the present invention relates to a method for diagnosing endometriosis, said method comprising obtaining a sample of menstrual blood measuring basal cells in said sample or measuring at least one gene product typical for cells of the basal endometrium in said sample and correlating said measurement to the diagnosis of endometriosis.

In a further preferred embodiment the present invention relates to a method for diagnosing endometriosis, said method comprising obtaining a sample of menstrual blood measuring at least one gene product typical for basal cells in said sample and correlating said measurement to the diagnosis of endometriosis.

As discussed above, several theories on the etiology of endometriosis and also a non-consistent terminology is present in the field.

In the sense of the present invention, "endometriosis" is meant to cover both the aspects which in the literature are known as endometriosis and adenomyosis.

As mentioned, the inventive method is based on a sample of menstrual blood. "Menstrual blood" in the sense of the present invention refers to the red blood cells containing fluids which during menses can be collected by various means from vaginal bleeding. In a preferred embodiment menstrual blood is directly collected from the posterior vaginal fornix. Other methods of collecting menstrual blood are the use of absorptives like pads and tampons and their appropriate extraction. It is also preferred to assess the menstrual blood derived in timed samples, i.e. to compare samples taken at the same day after onset of bleeding.

The present invention makes use of a "gene product" (especially a mRNA or a polypeptide) which is "typical for basal cells".

The skilled artisan, by routine procedures, can easily assess whether a gene product is typical for layer IV (i.e. found in layer IV in a significantly higher level as compared to cells of the functionalis ) or can be considered specific (present in layer IV and essentially no detectable level of the same gene product in cells of the functionalis. Quantitative routine measures to assess "typical" and "specific" are also at hand and, where considered necessary, may be used.

In the sense of the present invention a gene product is considered typical or a marker molecule for the basal endometrium if said gene product in basal cells is found in amounts at least twice as abundant or higher, or at most half as abundant or lower in basal cells as compared to cells of the other endometrial layers. More preferred the amount of said gene product is increased by at least 5-fold or even more preferred by at least 10-fold, as compared to the cells of the functionalis.

Most easily, both mRNA or polypeptide levels, respectively, are assessed by aid of radioactively labeled detection molecules and their level is assessed by quantifying the radio activity bound to cells of the functionalis as compared to cells of level IV of the endometrium.

The more pronounced the difference in expression pattern for a gene product, the more easy this gene product is used in the detection of basal cells.

It is therefore preferred to use in a method according to the present invention a gene product which is found in a ten-fold higher level in basal cells as compared to cells of the functionalis III of the endometrium. Gene products specific for basal cells are even more preferred.

The term "specific" implies that there is reactivity to one element and that there is essentially no reactivity to the second element for which specificity is assessed. In the present invention a marker is considered specific for basal cells, if it is present there and essentially absent from cells of the functionalis layer of the endometrium during menstruation. However, very low reactivity (< 1% functionalis /layer IV) does not exclude the use of the term specific and gene-products found in such low levels in endometrial cells of the functionalis as compared to layer IV cells, nonetheless, are considered basal cell specific. Preferably, the marker used is a specific marker and most preferred this marker is a specific marker which is only found in basal cells and essentially not found in cells of the functionalis of endometrium.

As the skilled artisan appreciates the diagnosis of a disease based on biochemical or immunological methods always is a compromise between the two extremes of 100 % sensitivity and 100 % specificity. This is especially known from the field of tumor markers. The situation for the present invention is quite similar. With the procedures according to the present invention it is now possible to correlate the measurement of basal cells or the measurement of a gene product typical for basal cells from a sample of menstrual blood to endometriosis.

The more abundant these cells or these gene products are found, the more likely a patient suffers from endometriosis.

In a preferred embodiment the gene product measured is a polypeptide. The following definitions shall apply in this context.

"Diagnostic" means identifying the presence or nature of a pathologic condition. Diagnostic methods differ in their specificity and sensitivity. While a particular diagnostic method may not provide a definitive diagnosis of a condition, it suffices if the method provides a positive indication that aids in diagnosis.

"Test amount" refers to an amount of an analyte in a subject sample, which is then compared to a normal amount of the analyte in a sample (e.g., from a healthy individual) such that the relative comparison of the values provides a reference value for diagnosing a designated disease. Depending upon the method of detection, the test amount may be a determination of the amount of the analyte, but it is not necessarily an amount. The test amount may also be a relative value, such as a plus or a minus score, and also includes an amount indicating the presence or absence of the analyte in a sample.

"Normal amount" refers to an amount or a range of an analyte in a biological sample that indicates health or lack of pathology.

"Diagnostic amount" refers to an absolute or normalized amount of an analyte in a biological sample that is consistent with the diagnosis of a particular disease.

"Polypeptide" refers to a polymer composed of amino acid residues, related naturally occurring structural variants, and synthetic non-naturally occurring analogs thereof linked via peptide bonds, related naturally occurring structural variants, and synthetic non-naturally occurring analogs thereof. Synthetic polypeptides can be synthesized, for example, using an automated polypeptide synthesizer.

"Affinity agent" refers to any material capable of adsorbing an analyte. This includes, without limitation, anion exchange materials, metal chelators, antibodies, protein ligands and polynucleotides.

"Antibody" refers to a polypeptide ligand substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and µ heavy chain constant region genes, and the myriad immunoglobulin variable region genes. Antibodies exist, e.g., as intact immunoglobulins or as a number of well characterized fragments produced by digestion with various peptidases. This includes, e.g., Fab' and F(ab)'₂ fragments. The term "antibody", as used herein, also includes antibody fragments either produced by the modification of whole antibodies or those synthesized *de novo* using recombinant DNA methodologies. It also includes polyclonal antibodies, monoclonal antibodies, chimeric antibodies and humanized antibodies.

A ligand or a receptor (e.g., an antibody) "specifically binds to" or "is specifically immunoreactive with" a compound analyte when the ligand or receptor functions in a binding reaction which is determinative of the presence of the analyte in a sample of heterogeneous compounds. Thus, under designed assay (e.g., immunoassay) conditions, the ligand or receptor binds preferentially to a particular analyte and does not bind in a significant amount to other compounds present in the sample.

"Immunoassay" refers to a method of detecting an analyte in a sample involving contacting the sample with an antibody that specifically binds to the analyte and detecting binding between the antibody and the analyte. A variety of immunoassay formats may be used to select antibodies specifically immunoreactive with a particular protein. For example, solid-phase ELISA immunoassays are routinely used to select monoclonal antibodies specifically immunoreactive with a protein. See Harlow and Lane, in "Antibodies, A Laboratory Manual" (1988), Cold Spring Harbor Publications, New York, for a description of immunoassay formats and conditions that can be used to determine specific immunoreactivity.

"Detecting" refers to determining the presence, absence, or amount of an analyte in a sample, and can include quantifying the amount of the analyte in a sample or per cell in a sample.

"Detectable moiety" or a "label" refers to a composition detectable by spectroscopic, photochemical, biochemical, immunochemical, or chemical means. For example, useful labels include ³²P, ³⁵S, fluorescent dyes, electron-dense reagents, enzymes (e.g., as commonly used in an ELISA), biotin-streptavidin, dioxigenin, haptens and proteins for which anti-sera or monoclonal antibodies are available, or nucleic acid molecules with a sequence complementary to a target. The detectable moiety often generates a measurable signal, such as a radioactive, chromogenic, or fluorescent signal, that can be used to quantitate the amount of bound detectable moiety in a sample.

The detectable moiety may be directly or indirectly detectable. Indirect detection can involve the binding of a second directly or indirectly detectable moiety to the detectable moiety. For example, the detectable moiety can be the ligand of a binding partner, such as biotin or a hapten (e.g., digoxin or digoxigenin), which is a binding partner for streptavidin or a corresponding anti-hapten antibody, respectively. The binding partner may itself be directly detectable, for example, an antibody may be itself labeled with a fluorescent molecule. The binding partner also may be indirectly detectable, for example a labeled secondary antibody.

"Linker" refers to a molecule that joins two other molecules, either covalently, or through ionic, van der Walls or hydrogen bonds. Linkers are often used to couple a label to a specific binding partner.

The methods of detecting a polypeptide are numerous, including for example mass spectroscopy, chromatographic procedures and a variety of immunological procedures. Amongst the immunological methods especially the so-called immunoassays, the immunohistological procedures and affinity capture mass spectroscopy shall be mentioned.

An immunoassay according to the present invention - used to detect a marker molecule derived from basal endometrial cells - employs at least one antibody reagent as defined above. Many different immunological procedures are suitable and the skilled artisan will find all information required for setting out and performing such assays in relevant text books, like Tijssen - in "Practice and theory of enzyme immunoassays" (1990), Amsterdam, Elsevier.

Typically, the specific antibody reagent is directly or indirectly coated to a solid support, like a membrane (i. g., nitrocellulose) a micro-titer-plate (manufactured for example from PVC, polypropylene or polystyrene), test-tube (glass or a plastic material as described by micro-titer-plates), a dip-stick (manufactured from the same material as manufactured for test-tube) or a particulate material which usually is based on latex or polystyrene.

For direct coating the antibody is adsorbed to the solid phase directly as the name says. This, however, in many cases has proven disadvantageous, because part of the antibody thereby is denatured, only a rather low density is achieved and conditions for coating have to be optimized for each individual antibody reagent.

Many recently developed immunoassays therefore are based on indirect coating of antibodies to the solid phase. This coating is mediated by use of an analyte-independent binding pair. Well-known examples of such analyte-independent binding pairs are the biotin/streptavidin system or the digoxin-antidigoxin system. Details of such methods are especially given in US 5,858,803 and US 5,362,655.

For routine applications it is preferred to use a solid phase pre-coated with streptavidin and to indirectly bind the analyte specific component of the assay system(e.g. an antibody) to such solid phase in its biotinylated form.

Two types of assay setups are routinely used in analyte detection procedures which are generally referred to competitive-type and sandwich-type assays, respectively. Different ways are known from the literature to perform for example a competitive-type assay. In one mode, the analyte to be detected is labeled and competes with the analyte contained in the sample to be investigated. The more analyte present in the test sample, the lower the signal found in such a competitive-type assay.

When using a sandwich-type assay, both sides of such sandwich are formed by a specific binding partner to the analyte under investigation, preferably both are antibodies. Appropriate antibody reagents are selected which mutually do not interfere in their respective binding to the analyte. Such antibodies are modified to allow for solid phase binding or for signal generation. In such a sandwich-type assay the signal generated is directly proportional to the amount of analyte present in the sample.

In a further preferred embodiment the diagnostic method according to the present invention is characterized in that the detection is performed by immunohistochemistry. Whereas in the immunoassay procedures as described above, the cells contained in a menstrual blood sample are solubilized, for immunohistochemical procedures the menstrual blood sample is directly transferred into a phosphate buffer saline solution which contains formaldehyde. The cells and tissue fragments contained in the menstrual blood are mixed and fixed therein, allowed to settle, embedded according to standard procedures, and sections cut therefrom are investigated in immunohistochemistry. Several specific examples for performing such immunohistochemical procedures are given in the Examples section.

In immunohistochemistry the investigator has several criteria at hand, for example tissue morphology as well as staining intensity. The skilled pathologist based on the criteria available can easily tell whether a sample analyzed does contain basal cells or not. For such staining procedures polypeptides typical for basal cells i. e. leading to a stronger staining of basal cells, are sufficient for a positive diagnosis of such cells.

It can also be envisaged, that the cells and tissue fragments as contained in menstrual blood are treated to release individual cells from tissue fragments. Such single cells can easily be stained according to standard procedures and analyzed by a fluorescence activated cell sorter.

Using immunohistochemistry on samples from biopsies (obtained by hysterectomy) it has been found that at the time of bleeding only basal cells layer IV of the human endometrium contain significant amounts of estrogen receptor, progesterone receptor, especially progesterone receptor isoform A and aromatase p450. At the time of menses these markers are confined to and specific for cells of the basal endometrium. In a preferred embodiment the polypeptide detected is therefore selected from the group consisting of estrogen receptor, progesterone receptor, progesterone receptor type A, and p450 aromatase. Since the staining for p450 aromatase is most intensive and progesterone receptor type A only has been found in basal cells of the endometrium and not in other layers of the endometrium it is preferred to select either progesterone receptor type A or p450 aromatase. Most preferred is the detection of p450 aromatase.

In a further embodiment the "gene product" is an mRNA. Of course, in diagnostic procedures, a corresponding c-DNA may be used. The following definitions shall apply.

"Nucleic acid sequence", "nucleotide sequence" and "polynucleotide sequence" as used herein refer to an oligonucleotide or polynucleotide, and fragments or portions thereof, and to DNA or RNA of genomic or synthetic origin which may be single- or double-stranded, and represent the sense or antisense strand.

As used herein, the terms "oligonucleotides" and "oligomers" refer to a nucleic acid sequence of at least about 10 nucleotides and as many as about 60 nucleotides, preferably about 15 to 30 nucleotides, and more preferably about 20 - 25 nucleotides, which can be used as a probe or amplimer.

As used herein, the terms "complementary" or "complementarity" are used in reference to "polynucleotides" and "oligonucleotides" (which are interchangeable terms that refer to a sequence of nucleotides) related by the base complementary to the sequence "5'-ACTG-3'." Complementarity can be "partial" or "total". "Partial" complementarity is where one or more nucleic acid bases is not matched according to the base pairing rules. "Total" or "complete" complementarity between nucleic acids is where each and every nucleic acid base is matched with another base under the base pairing rules. The degree of complementarity between nucleic acid strands has significant effects on the efficiency and strength of hybridization between nucleic acid strands. This is of particular importance in amplification reactions, as well as detection methods which depend upon binding between nucleic acids.

As used herein, the term "hybridization" is used in reference to the pairing of complementary nucleic acids using any process by which a strand of nucleic acid joins with a complementary strand through base pairing to forma hybridization complex. Hybridization and the strength of hybridization (i.e., the strength of the association between the nucleic acids) is impacted by such factors as the degree of complementarity between the nucleic acids, stringency of the conditions involved, the Tₘ of the formed hybrid, and the G:C ratio within the nucleic acids.

As used in herein, the term "sample template" refers to nucleic acid originating from a sample which is analyzed for the presence of a target sequence of interest. In contrast, "background template" is used in reference to nucleic acid other than sample template which may or may not be present in a sample. Background template is most often inadvertent. It may be the result of carryover, or it may be due to the presence of nucleic acid contaminants sought to be purified away from the sample. For example, nucleic acid from organisms other than those to be detected may be present as background in a test sample.

"Amplification" is defined as the production of additional copies of a nucleic acid sequence and is generally carried out using polymerase chain reaction technologies well known in the art ("PCR Primer, a Laboratory Manual" (1995), Eds. C. W. Dieffenbach and G. S. Dveksler, Cold Spring Harbor Press, Plainview N.Y.). As used herein, the term "polymerase chain reaction" ("PCR") refers to the method of K. B. Mullis US 4,683,195 and US 4,683,202, hereby incorporated by reference, which describe a method for increasing the concentration of a segment of a target sequence in a mixture of genomic DNA without cloning or purification. The length of the amplified segment of the desired target sequence is determined by the relative positions of two oligonucleotide primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified".

With PCR, it is possible to amplify a single copy of a specific target sequence in genomic DNA to a level detectable by several different methodologies (e.g., hybridization with a labeled probe; incorporation of biotinylated primers followed by avidin-enzyme conjugate detection; incorporation of ³²P-labeled deoxynucleotide triphosphates, such as dCTP or dATP, into the amplified segment). In addition to genomic DNA, any oligonucleotide sequence can be amplified with the appropriate set of primer molecules. In particular, the amplified segments created by the PCR process itself are, themselves, efficient templates for subsequent PCR amplifications.

The term "reverse transcription polymerase chain reaction" and "RT-PCR" refer to a method for reverse transcription of an RNA sequence to generate a mixture of cDNA sequences, followed by increasing the concentration of a desired segment of the transcribed cDNA sequences in the mixture without cloning or purification. Typically, messenger RNA (mRNA) is reverse transcribed using a single primer (e.g., an oligo-dT primer) prior to PCR amplification of the desired segment of the transcribed DNA using two primers.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (i.e., in the presence of nucleotides and of an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (i.e., a sequence of nucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule", so that it is detectable in any detection system, including, but not limited to enzyme, fluorescent, radioactive, and luminescent systems.

As used herein the term "coding region" when used in reference to a structural gene refers to the nucleotide sequences which encode the amino acids found in the nascent polypeptide as a result of translation of a mRNA molecule. The coding region is bounded, in eukaryotes, on the 5' side by the nucleotide triplet "ATG" which encodes the initiator methionine and on the 3' side by one of the three triplets which specify stop codons (i.e., TAA, TAG, TGA).

As used herein, the term "structural gene" or "structural nucleotide sequences" refers to a DNA sequence coding for RNA or a protein which does not control the expression of other genes. In contrast, a "regulatory gene" or "regulatory sequence" is a gene which encodes products (e.g. transcription factors) which control the expression of other genes.

As used herein, the term "gene" means the deoxyribonucleotide sequences comprising the coding region of a structural gene. A "gene" may also include non-translated sequences located adjacent to the coding region on both the 5' and 3' ends such that the gene corresponds to the length of the full-length mRNA. The sequences which are located 5' of the coding region and which are present on the mRNA are referred to as 5' non-translated sequences. The sequences which are located 3' or downstream of the coding region and which are present on the mRNA are referred to as 3' non-translated sequences. The term "gene" encompasses both cDNA and genomic forms of a gene. A genomic form or clone of a gene contains the coding region interrupted with non-coding sequences termed "introns" or "intervening regions" or "intervening sequences". Introns are segments of a gene which are transcribed into heterogenous nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

In a preferred mode of the present invention the mRNA level of a gene typically expressed in basal cells is assessed. Most preferred an mRNA corresponding to a gene specifically expressed in layer IV cells as compared to cells of endometrial epithelium is assessed.

Preferably, the basal cell m-RNA is the transcript of a gene coding for estrogen receptor (ER), progesterone receptor type A (PR-A), or aromatase p450. Most preferred are mRNAs of progesterone receptor type A and aromatase p450.

Such mRNA measurement is performed according to standard and routine procedures, such methods involve detecting and/or quantifying expression of a marker gene for basal cells mRNA or cDNA using amplification-based assays with or without signal amplification, hybridization based assays, and combination amplification-hybridization assays.

Polynucleotide assays preferably are performed with a sample of nucleic acid isolated from the biological sample.

The polynucleotide (e.g., genomic DNA, RNA or cDNA) may be isolated from the sample according to any of a number of methods well known to those of skill in the art. Methods for isolating nucleic acids are well known to those of kill in the art and are described, for example, in Tijssen, P., Hybridization with nucleic acid probes Part I: Theory and nucleic acid preparation in "Laboratory Techniques in Biochemistry and Molecular Biology, Vo" (1993); Seite 19 WO 99/63116; Sambrook, J., et al. in "Molecular Cloning: A Laboratory Manual" (1989), Cold Spring Harbour, NY, Cold Spring Harbour Laboratory Press; Ausubel, F., et al. - in "Current protocols in molecular biology" (1994), Wiley Verlag.

In one embodiment, the assays of the present invention are amplification-based assays for detection of a gene product typical for basal cells (e.g., mRNA or cDNA; hereinafter also referred to as "target"). In an amplification based assay, all or part of an polynucleotide target is amplified, and the amplification product is then detected directly or indirectly. This includes detecting the amplification product by hybridization with a probe, by detecting a product of the appropriate size on a gel or by mass spectrometry, for example, of by determining the sequence of at least a part of the amplification product. When there is no underlying gene product to act as a template, no amplification product is produced (e.g., of the expected size), or amplification is non-specific and typically there is no single amplification product. In contrast, when the underlying gene or gene product is present, the target sequence is amplified, providing an indication of the presence and/or quantity of the underlying gene or mRNA. Target amplification-based assays are well known to those of skill in the art.

Primers and probes for detecting gene product typical for basal cells may be designed and produced by those of skill by referring to the sequences of genes which are expressed in a pattern typical for layer IV of the endometrium. Suitable primers and probes are sufficiently complementary to the gene product typical for basal cells to hybridize to the target nucleic acid. Primers are usually between about 10 and about 100 bases, typically between about 12 and about 50 bases, and may amplify all, or any portion, of the gene product typical for basal cells.

In one embodiment, a gene product typical for basal cells is amplified and detected using the polymerase chain reaction (including all variants, e.g., reverse-transcriptase-PCR; the Sunrise Amplification System (Oncor, Inc, Gaithersburg MD); and numerous others known in the art). In one illustrative embodiment, PCR amplification is carried out in a 50 µl solution containing the nucleic acid sample (e.g., cDNA obtained through reverse transcription of mRNA), 100 µM in each dNTP (dATP, dCTP. dGTP and dTTP; Pharmacia LKB Biotechnology, NJ), the mRNA-specific PCR primer(s), 1 unit/ Taq polymerase (Perkin Elmer, Norwalk CT), 1 x PCR buffer (50 mM KC1, 10 mM Tris, pH 8.3 at room temperature, 1.5 mM MgCI₂, 0.01% gelatin) with the amplification run for about 30 cycles at 94° for 45 sec, 55° for 45 sec and 72° for 90 sec.

However, as will be appreciated, numerous variations may be made to optimize the PCR amplification for any particular reaction. Other suitable target amplification methods include the ligase chain reaction (LCR; e.g., Wu, D. Y. and Wallace, R. B., Genomics 4 (1989) 560-9; Landegren, U., et al., Science 241 (1988) 1077-80; Barany, F., Proc Natl Acad Sci U S A 88 (1991) 189-93; and Barringer, K. J., et al., Gene 89 (1990) 117-22); strand displacement amplification (SDA; e.g., Walker, G. T., et al., Proc Natl Acad Sci U S A 89 (1992) 392-6); transcription amplification (e.g., Kwoh, D. Y., et al., Proc Natl Acad Sci U S A 86 (1989) 1173-7); self-sustained sequence replication (3SR; e.g., Fahy, E., et al., PCR Methods Appl 1 (1991) 25-33; Guatelli, J. C., et al., Proc Natl Acad Sci U S A 87 (1990) 1874-8); the nucleic acid sequence based amplification (NASBA, Cangene, Mississauga, Ontario; e.g., Compton, J., Nature 350 (1991) 91-2); the transcription-based amplification System (TAS); and the self-sustained sequence replication System (SSR).

One useful variant of PCR is PCR ELISA (e.g., Boehringer Mannheim Cat. No. 1636 111) in which digoxigenin-dUTP is incorporated into the PCR product. The PCR reaction mixture is denatured and hybridized with a biotin-labeled oligonucleotide designed to anneal to an internal sequence of the PCR product. The hybridization products are immobilized on streptavidin coated plates and detected using anti-digoxigenin antibodies. Examples of techniques sufficient to direct persons of skill through *in vitro* amplification methods are found in PCR Technology: Principles and Applications for DNA Amplification (1992), Eds. H. Erlich, Freemann Press, New York; PCR protocols: A guide to Methods and Applications (1990), Eds. Innis, Gelfand, Snisky and White, Academic Press, San Diego.

Amplified products may be directly analyzed, e.g., by size as determined by gel electrophoresis; by hybridization to a target nucleic acid immobilized on a solid support such as a bead, membrane, slide, or chip; by sequencing; immunologically, e.g., by PCR-ELISA, by detection of a fluorescent, phosphorescent, or radioactive signal; or by any of a variety of other well-known means. An illustrative example of a detection method uses PCR primers augmented with hairpin loops linked to fluorescein and a benzoic acid derivative that serves as a quencher, such that fluorescence is emitted only when the primers unfold to bind their targets and replication occurs.

In addition, methods known to increase the signal produced by amplification of the target sequence may be used. Methods for augmenting the ability to detect the amplified target include signal amplification system such as: branched DNA signal amplification (e.g., US 5,124,246; Urdea, M. S., Biotechnology (N Y) 12 (1994) 926-8); tyramide signal amplification (TSA) System (DuPont); catalytic signal amplification (CSA; Dako); Q Beta Replicase Systems (Tyagi, S., et al., Proc Natl Acad Sci U S A 93 (1996) 5395-400).

One of skill in the art will appreciate that whatever amplification method is used, a variety of quantitative methods known in the art can be used if quantitation is desired. Detailed protocols for quantitative PCR may be found in PCR protocols: A guide to Methods and Applications (1990), Eds. Innis, Gelfand, Snisky and White, Academic Press, San Diego and Ausubet et al., supra (Unit 15) and Diaco, R., Practical Considerations for the Design of Quantitative PCR Assays in "PCR STRATEGIES" (1995) 84-108, Eds. I. e. al., Academic Press, New York; US 5,629,154.

A variety of methods for specific DNA and RNA measurement using nucleic acid hybridization techniques are known to those of skill in the art (see Sambrook et al., supra).

Hybridization based assays refer to assays in which a probe nucleic acid is hybridized to a target nucleic acid. Methods of selecting nucleic acid probe sequences for use in nucleic acid hybridization are discussed in Sambrook et al., supra and are based on the gene sequence of any appropriate gene typical or preferably specific for basal cells. In some formats, at least one of the target and probe is immobilized. The immobilized nucleic acid may be DNA, RNA, or another oligo- or poly-nucleotide, and may comprise natural or non-naturally occurring nucleotides, nucleotide analogs, or backbones. Such assays may be in any of several formats including: Southern, Northern, dot and slot blots, high-density polynucleotide or oligonucleotide arrays (e.g., Genechips™ Affymetrix), dip sticks, pins, chips, or beads. All of these techniques are well known in the art and are the basis of many commercially available diagnostic kits. Hybridization techniques are generally described in "Nucleic Acid Hybridization, A practical approach" (1985), Eds. Hames; Gall, J. G. and Pardue, M. L., Proc Natl Acad Sci U S A 63 (1969) 378-83; John, H. A., et al., Nature 223 (1969) 582-7.

One common format is direct hybridization, in which a target nucleic acid is hybridized to a labeled, complementary probe. Typically, labeled nucleic acids are used for hybridization, with the label providing the detectable signal. One method for evaluating the presence, absence, or quantity of a target mRNA is carrying out a Northern transfer of RNA from a sample and hybridization of a labeled target-specific nucleic acid probe. Other common hybridization formats include sandwich assays and competition or displacement assays. Sandwich assays are commercially useful hybridization assays for detecting or isolating nucleic acid sequences. Such assays utilize a "capture" nucleic acid covalently immobilized to a solid support and a labeled "signal" nucleic acid in solution. The biological or clinical sample will provide the target nucleic acid. The "capture" nucleic acid and "signal" nucleic acid probe hybridize with the target nucleic acid to form a "sandwich" hybridization complex. In the absence of target nucleic acid the signal nucleic acid cannot hybridize with the capture nucleic acid.

The present invention also provides probe-based hybridization assays for gene product typical for basal cells employing arrays of immobilized oligonucleotide or polynucleotides to which a target nucleic acid can hybridize. High density oligonucleotide arrays or polynucleotide arrays provide a means for efficiently detecting the presence and characteristics (e.g., sequence) of a target nucleic acid (e.g., a mRNA of estrogen receptor progesterone receptor type A, or aromatase p450). Techniques are known for producing arrays containing thousands of oligonucleotides complementary to defined sequences, at defined locations on a surface using photolithographic techniques for synthesis *in situ* (see, e.g., US 5,578,832; US 5,556,752; and US 5,510,270; Fodor, S. P., et al., Science 251 (1991) 767-73; Pease, A. C., et al., Proc Natl Acad Sci U S A 91 (1994) 5022-6; and Lockhart, D. J., et al., Nat Biotechnol 14 (1996) 1675-80) or other methods for rapid synthesis and deposition of defined oligonucleotides (Blanchard, A. P., et al., Biosensors & Bioelectronics 11 (1996) 687-690). When these methods are used, oligonucleotides (e.g., 20-mers) of known sequence are synthesized directly on a surface such as a derivatized glass slide. Usually, the array produced is redundant, having several oligonucleotide probes on the chip specific for the polynucleotide corresponding to the gene typical for basal cell to be detected.

An alternative means for detecting expression of a gene encoding a protein typical for basal cells is *in situ* hybridization. *In situ* hybridization assays are well known and are generally described in Angerer, L. M., et al., Methods Enzymol 152 (1987) 649-61 and Ausubel et al., *supra.* In an *in situ* hybridization assay, cells or tissue specimens are fixed to a solid support, typically in a permeabilized state, typically on a glass slide. The cells are then contacted with a hybridization solution at a moderate temperature to permit annealing of labeled nucleic acid probes (e.g., "S-labeled riboprobes, fluorescently labeled probes) completely or substantially complementary to the target mRNA. Free probe is removed by washing and/or nuclease digestion, and bound probe is visualized directly on the slide by autoradiography or an appropriate imaging techniques, as is known in the art.

In alternative embodiments of polynucleotide assays it is not necessary to isolate nucleic acids (e.g., total or polyA⁺ RNA) from the biological sample prior to carrying out amplification, hybridization or other assays. These embodiments have certain advantages when RNA is to be measured, because they reduce the possibility of loss of mRNA during isolation and handling. For example, many amplification techniques such as PCR and RT-PCR defined above can be carried out using permeabilized cells (histological specimens and FACS analyses), whole lysed cells, or crude cell fractions such as certain cell extracts. Preferably, steps are taken to preserve the integrity of the target nucleic acid (e.g., mRNA) if necessary (e.g., addition of RNAase inhibitors). Amplification and hybridization assays can also be carried out *in situ*. according to the present invention in thin tissue sections from a sample of menstrual blood cells after fixation and embedding such cells (this way of preparing a sample as described in detail in Example 1) PCR, RT-PCR, or LCR amplification methods may be carrier out, as well as known in the art, *in situ,* e.g., using a polymerase or ligase, a primer or primer(s), and (deoxy)ribonucleoside triphosphates (if a polymerase is employed), and reverse transcriptase and primer (if RNA is to be transcribed and the cDNA is to be detected) on fixed, permeabilized, or microinjected cells to amplify target RNA. This method is often useful when fluorescently-labeled dNTPs, primers, or other components are used in conjunction with microscopy. In regard to their diagnostic utility, the results obtained by *in situ* PCR resemble those obtained by immunohistochemistry.

It is also a preferred embodiment according to the present invention to measure between 2 and 5 marker gene products of basal cells in order to further enhance the diagnostic sensibility and/or specificity and to even better correlate the values thus measured from a sample of menstrual blood to endometriosis.

Yet a further diagnostic improvement can be achieved if the value measured for a gene product typical for basal cells is "normalized" to a gene product "from cells of the functionalis". Such gene product is present in the cells of the functionalis of the endometrium in rather a constant amount throughout the menstrual cycle, or such gene product is at least rather constant during menses. Such gene product is for example derived from a gene selected from a gene known in the art as house-keeping gene. Typical house-keeping genes are the genes coding for proteins of the cytoskeleton.

In a preferred embodiment the present invention describes a method not requiring the presence of intact basal cells. Rather the method for diagnosing endometriosis comprises obtaining a sample of menstrual blood, measuring at least one gene product typical for cells of the basal endometrium in said sample and correlating the measurement to the diagnosis of endometriosis. In order to perform such measurement, it is preferred to treat the menstrual blood sample by appropriate measures in order to release the gene products from cellular constituents. As a result, the gene product(s) of interest is obtained in soluble and easily accessible form. In a preferred mode, the sample is diluted into an appropriate incubation buffer. Such incubation buffers are well-known to the skilled artisan. In case of immunoassays an appropriate buffer is selected, serving both the purposes to liberate and/or solubilize proteins and to allow for immunological binding and thus for formation of an immunological complex between the target protein and the immunological binding partner used.

In a further preferred embodiment, the present invention relates to a method for diagnosing endometriosis, said method comprising obtaining a sample of menstrual blood measuring the amount of at least one gene product typical for basal cells in said sample, measuring the amount of a gene product from cells of the functionalis of the endometrium in said sample, assessing the relative amount of the gene product typical for basal cells to the gene product from cells of the functionalis and correlating said relative amount to endometriosis.

A gene product which is typical for cells of the functionalis, especially at the time of menses is for example 17βHSD-type II(HSD = hydrosteroide dehydrogenase).

Preferably, the gene product level or the gene product levels measured for gene products typical for basal cells are expressed relative to the gene product level of gene coding for a cytoskeletal protein or 17βHSD-type II. Most preferred 17βHSD is used to normalize the gene product typical or specific for basal cells and thus to correct for variations, e.g., induced by sampling mode, time or by the intensity of bleeding.

Most preferred a gene product which is specific for basal cells is normalized to a house-keeping gene of cells from the functionalis of the endometrium .

The following examples and references are provided to aid the understanding of the present invention, the true scope of which is set forth in the appended claims. It is understood that modifications can be made in the procedures set forth without departing from the spirit of the invention.

### Examples

### Example 1: Immunohistology form samples of tissue and menstrual blood

### 1.1 Tissue samples

Non-pregnant uteri were obtained from 70 pre-menopausal women (aged 24 - 55 years) undergoing hysterectomy for various reasons. Of these women 51 were suffering from endometriosis and/or adenomyosis (aged 26-55 years, mean 42 years). The uteri of the remaining 19 women (aged 24 - 49 years, mean 36 years) were devoid of endometrial and myometrial pathology and during laparotomy of these women there were no signs of endometriosis. All women had regular cycles and no history of hormone therapy for at least six months. The respective phases of the cycle were ensured by correlating the date of the last menstrual period with histological findings according to the usual histological dating method (Dallenbach-Hellweg, G. and Poulsen, H., in "Atlas der Histophathologie des Endometriums" (1984, Springer Verlag Berlin, Heidelberg, New York, Tokyo).

In addition, a total number of 35 endometriotic lesions were obtained via laparoscopy or laparotomy from normally cycling pre-menopausal women (aged 25 - 51 years, mean 35 years) without hormone therapy for at least six months. These specimen were excised from various intra- and extra-peritoneal locations such as the parietal, intestinal, uterine, urinary bladder peritoneum (n=25), ovarian endometriomas (n=6) and the recto-vaginal septum (n=4), respectively. In all cases the exact phase of cycle was known. Table I is a compilation of the number of tissue samples with respect to origin, menstrual phase and immunohistochemistry performed, respectively.

**Table I**

| | uteri of women with endometriosis | | | adenomyotic lesions | | | endometriotic lesions | | | uteri of healthy controls | | |
|---|---|---|---|---|---|---|---|---|---|---|---|---|
| phase of cycle (days) | endometrium | | myometrium | epithelium/ stroma | | PMC | epithelium/ stroma | | PMC | endometrium | | myometrium. |
| early profit. phase (4-7) | 10 *(15)* | | 10 | 10 *(15)* | | 10 | 8 *(9)* | | 8 | 4 *(5)* | | 4 |
| mid-prolif. phase (8-11) | 6 *(5)* | ***8*** | 6 | 6 *(5)* | ***7*** | 6 | 9 *(9)* | ***13*** | 9 | 2 *(2)* | ***4*** | 2 |
| late prolif. phase. (12-14) | 5 *(8)* | | 4 | 3 *(8)* | | 3 | 5 *(5)* | | 3 | 2 *(1)* | | 2 |
| early secret. Phase (15-18) | 4 *(5)* | | 4 | 4 *(5)* | | 4 | 3 *(3)* | | 3 | 3 *(2)* | | 3 |
| mid-secret. phase (19-23) | 6 *(8)* | ***10*** | 6 | 6 *(8)* | ***9*** | 6 | 5 *(4)* | ***10*** | 2 | 3 *(4)* | ***5*** | 3 |
| late secret. phase (24-28) | 6 (6) | | 5 | 5 (6) | | 5 | 5 (5) | | 3 | 3 (5) | | 3 |
| menstrual phase (1-3) | 3 *(4)* | | 3 | 3 *(4)* | | 3 | | | | | | |
| total | 40 *(51)* | ***1*** ***8*** | 38 | 37 *(51)* | ***16*** | *37* | 35 *(35)* | ***23*** | 28 | 17 *(19)* | ***9*** | 17 |

The hysterectomy specimens and the excised endometriotic lesions were fixed with 4% buffered formaldehyde (pH 7.1) for twenty four hours.

Sagitally oriented sections of the uterine corpus in the median line were taken from the fundal region and from the anterior and posterior wall containing the entire uterine wall from the uterine cavity to the serosa. One half of the uterine corpus was cut into at least seven slices starting from the tubal isthmus along the intramural passage of the tube (orthogonally with respect to the endometrial surface) to identify adenomyotic and subserosal endometriotic lesions, respectively.

The uterine tissue sections and the excised endometriotic lesions were embedded in paraffin.

### 1.2 Menstrual blood samples

A total number of 43 menstrual blood samples were collected from 19 healthy women (aged 25 - 37 years, mean 33 years) and 24 women (aged 24 - 42, mean 33 years) years with endometriosis. All women included in this part of the study had undergone laparoscopy and in cases of endometriosis staging of the disease was performed according to the revised AFS classification of the American Society for Reproductive Medicine (13, 5, 3 and 3 patients were stage 1, 2, 3 and 4 respectively). All women had regular cycles and no history of hormone therapy for at least six months. They came into our unit in the morning of their second day of menstruation after having used just a sanitary pad during the foregoing night. In all women roughly one milliliter of menstrual blood was aspirated from the posterior vaginal fornix into a syringe pre-filled with two milliliter of 4% buffered formaldehyde (pH 7.1) for fixation.

Before embedding in paraffin the menstrual blood samples were washed with NaCl-solution to remove red blood cells.

A series of four sequential sections of the endometrial fragments derived from the menstrual blood specimen were examined for the presence or absence of fragments that stained for estrogen receptor (ER), progesterone receptor (PR), progesterone receptor B isoform (PR-B) and p450 aromatase expression, respectively. The evaluation for each of the individual markers was based on the examination of only one section of the menstrual specimen per marker.

### 1.3 Immunostaining

All immunohistochemical procedures were performed by using an automated slide stainer (Ventana NexES IHC Staining System; Ventana Medical Systems Inc., Tucson, AZ, USA). Paraffin-embedded sections (thickness 3 µm) were mounted on slides before deparaffination and rehydration.

### Oestrogen receptor (ER), progesterone receptor (PR) and progesterone receptor B isoform (PRB)

Unmasking of antigen was carried out by using a microwave oven for 40 minutes at 200W (Tris-EDTA-citrat buffer pH 7.8). The sections were then transferred to the Ventana NexES IHC Staining System. For receptor staining the following antibodies were used: Anti-oestrogen-receptor mouse monoclonal antibody developed against prokaryotic recombinant protein corresponding to the full-length alpha form of the oestrogen receptor molecule, at a dilution of 1:10 (Novocastra Laboratories, Newcastle upon Tyne, UK); anti-progesterone-receptor mouse monoclonal antibody developed against synthetic peptide corresponding to a site of predicted high antigenicity on the human progesterone receptor and binding to both known types of nuclear progesterone receptors (PR-A and PR-B; Viville, B., et al., Hum Reprod 12 (1997) 815-22) at a dilution of 1:10 (Novocastra Laboratories, Newcastle upon Tyne, UK); anti-progesterone-receptor-isoform B mouse monoclonal antibody developed against the progesterone receptor isoform B (PRB) at a dilution of 1:5 (Santa Cruz Biotechnology, Inc, USA). In the immunohistochemistry of PRB an amplification kit (Ventana Medical Systems Inc., Tucson, AZ, USA) was used for the enhancement of the staining. The automated staining procedure that included a Basic DAB Detection Kit was terminated by haematoxylin counterstaining and washing with water. The slides were then dehydrated and mounted.

Sections of receptor-positive mammary carcinomas served as positive controls. Negative controls were made using sections of receptor-positive mammary carcinomas without incubation with primary antibody and several oestrogen-receptor-negative tissues (e.g. from tonsils and non-gynecological carcinomas) using the same automated procedure.

### P450 aromatase (P450A)

The unmasking of the antigen and the following automated staining procedure that ended with the counterstaining with haematoxylin were the same except of using a Basic Alkaline Phosphatase Red Detection Kit. The staining was performed with primary rabbit anti-aromatase cytochrome P-450 polyclonal antibody recognizing human placental cytochrome P450 aromatase at a dilution of 1:1000 in ventana antibody diluent (Hauptman-Woodward Medical Research Inst., Inc., Buffalo, New York). Sections of normal ovarian tissue containing Graafian follicles served as positive controls. Several P450A-negative tissues (e.g. from tonsils and non-gynecological carcinomas) served as negative controls.

### Actin

Unmasking of antigen was perfumed by a protease solution (Ventana Medical Systems Inc., Tucson, AZ, USA) followed by the same staining-procedure as described for P450A. The primary anti-muscle actin antibody (clone HUC 1-1) was provided by Ventana Medical Systems Inc. in a pre-diluted form and staining performed as described for PR above.

The specific actin immunostaining was used to identify peristromal smooth muscle cells in endometriotic and adenomyotic lesions thereby facilitating the evaluation of their ER and PR staining.

### 1.4 Evaluation of staining intensity

### Oestrogen receptor (ER), progesterone receptor (PR)

Results of specific nuclear staining were evaluated using a semi-quantitative method. The immunoreactive score (IRS) was calculated using the following equitation: IRS=ΣPi (i+1), where i=1,2 or 3, and Pi is the percentage of stained cells for each intensity, according to the method described by Lessey, B. A., et al., J Clin Endocrinol Metab 67 (1988) 334-40. The staining intensity was graded as 0 = no, 1 = weak, 2 = moderate and 3 = strong staining, respectively.

The following tissues were counted out separately: The endometrial epithelium and stroma of the functionalis as represented by zone II and of the deep basalis as represented by zone IV; epithelium and stroma of endometriotic and adenomyotic lesions; the outer myometrium represented by the stratum vasculare of the neometra; the archimyometrium (stratum subvasculare); the peristromal muscular tissue of endometriotic and adenomyotic lesions. With respect to the uterine tissues at least 20 high power fields and with respect to the endometriotic lesions at least 5 high power fields were selected at random and evaluated. The immunoreactive score of positively stained cells per uterine region was determined by taking the arithmetic mean of the values of all counted high power fields. There was a high reproducibility of the method in that, in a test series, the interassay variation over the full range of IRS never exceeded 10%.

The data obtained from individual uterine specimen and endometriotic lesions of the premenopausal women were grouped according to phase of the menstrual cycle as described above.

### P450 aromatase (p450A) and progesterone receptor B isoform (PRB)

Results of specific staining were obtained using a semiquantitative method based on a four-point scale: 0 = no, 1 = weak, 2 = moderate and 3 = strong staining, respectively. The evaluation was confined to the eutopic and ectopic endometrium and used the same number of high power fields as described above.

### Statistical analysis

Statistical analysis was performed with Student's t-test for unpaired data and Fischer's test. Significance was assumed when p < 0.05.

### 1.5 Results

**Table III**

| parameter | healthy controls | | endometriosis | | significance (Fischer's-Test) |
|---|---|---|---|---|---|
| | positive/n | % | positive/n | % | |
| ER (epithelium and stroma) | 2/19 | 10 | 18/24 | 75 | p<0.00005 |
| PR (epithelium) | 0/19 | 0 | 8/24 | 33 | p<0.005 |
| PR (stroma) | 2/19 | 10 | 14/24 | 58 | p<0.000005 |
| PRB (epithelium and stroma) | 0/19 | 0 | 0/24 | 0 | not significant |
| P450 aromatase (epithelium and stroma) | 2/19 | 10 | 20/24 | 83 | p<0.000005 |

It becomes readily obvious from Table II that especially estrogen receptor and p450 aromatase are valuable markers for correlating the presence of basal cells in a menstrual blood sample to endometriosis.

### Example 2: Immunoassays from vaginal blood samples

### 2.1 Menstrual blood samples

Samples of menstrual blood from women with endometriosis as well as from women without endometriosis were collected in the morning of their second day of menstruation after having used just a sanitary pad during the foregoing night. From all women roughly one milliliter of menstrual blood was aspirated from the posterior vaginal fornix into a syringe pre-filled with two milliliter of phosphate buffered saline (PBS; pH 7.4).

### 2.2 Competitive immunoassay for progesterone receptor

Microtiter plates pre-coated with streptavidin (Roche Diagnostics GmbH, product number 1965905) were incubated with 100 µl of biotinylated monoclonal antibody to PR (Viville et al., *supra*) at a concentration of 100 ng/ml for 60' at room temperature. Vaginal blood samples were diluted 1:100 in PBS/Tween® 20 (0,05 or Tween) and 199 µl of such sample added per well. After washing to each well 100 µl of the synthetic peptide (as described by Viville et al., *supra*) labeled with peroxidase according to standard procedures) was added and incubation performed for 1 h at room temperature.

Wells again were washed (3 x 200 µl PBS/Tween) and the amount of peroxidase bound to the monoclonal antibody was assessed using ABTS® (ABTS stands for: 2,2'-azino-di[3-ethylbenzthiazoline sulfonate; Roche Biochemicals, Order no. 1 684 302) as a substrate according to the instructions given by the manufacturer.

### 2.3 Results

A significant level of progesterone receptor was measured in 67 % of the samples collected from patients with endometriosis, whereas in only 6 % of the patients without endometriosis progesterone receptor was detected.

### List of References

Anaf, V., et al., Hum Reprod 15 (2000) 767-71
Angerer, L. M., et al., Methods Enzymol 152 (1987) 649-61
Ausubel, F., et al. in "Current protocols in molecular biology" (1994), Wiley Verlag
Barany, F., Proc Natl Acad Sci U S A 88 (1991) 189-93
Barringer, K. J., et al., Gene 89 (1990) 117-22
Blanchard, A. P., et al., Biosensors & Bioelectronics 11 (1996) 687-690
Compton, J., Nature 350 (1991) 91-2
Dallenbach-Hellweg, G. and Poulsen, H., in "Atlas der Histophathologie des Endometriums" (1984), Springer Verlag Berlin, Heidelberg, New York, Tokyo
Diaco, R., Practical Considerations for the Design of Quantitative PCR Assays in "PCR STRATEGIES" (1995) 84-108, Eds. I. e. al., Academic Press, New York
Fahy, E., et al., PCR Methods Appl 1 (1991) 25-33
Fodor, S. P., et al., Science 251 (1991) 767-73
Gall, J. G. and Pardue, M. L., Proc Natl Acad Sci U S A 63 (1969) 378-83
Guatelli, J. C., et al., Proc Natl Acad Sci U S A 87 (1990) 1874-8
Harlow and Lane, in "Antibodies, A Laboratory Manual" (1988), Cold Spring Harbor Publications, New York
John, H. A., et al., Nature 223 (1969) 582-7
Kunz, G., et al., Hum Reprod 15 (2000) 76-82
Kwoh, D. Y., et al., Proc Natl Acad Sci U S A 86 (1989) 1173-7
Landegren, U., et al., Science 241 (1988) 1077-80
Lessey, B. A., et al., J Clin Endocrinol Metab 67 (1988) 334-40
Leyendecker, G., et al., Hum Reprod 11 (1996) 1542-51
Leyendecker, G., et al., Hum Reprod Update 4 (1998) 752-62
Leyendecker, G., Hum Reprod 15 (2000) 4-7
Lockhart, D. J., et al., Nat Biotechnol 14 (1996) 1675-80
Meyer, R., Zbl Gynäk. 43 (1919) 745-750
Noe, M., et al., Hum Reprod 14 (1999) 190-7
Okulicz, W. C., et al., Biol Reprod 49 (1993) 24-32
Padykula, H. A., et al., Biol Reprod 40 (1989) 681-90
PCR Primer, a Laboratory Manual (1995) -, Eds. C. W. Dieffenbach and G. S. Dveksler, Cold Spring Harbor Press, Plainview N.Y
PCR protocols: A guide to Methods and Applications (1990), Eds. Innis, Gelfand, Snisky and White, Academic Press, San Diego
PCR Technology: Principles and Applications for DNA Amplification (1992), Eds. H. Erlich, Freemann Press, New York
Pease, A. C., et al., Proc Natl Acad Sci U S A 91 (1994) 5022-6
Sambrook, J., et al. in "Molecular Cloning: A Laboratory Manual" (1989), Cold Spring Harbour, NY, Cold Spring Harbour Laboratory Press
Sampson, J. A., Am. J. Obstet. Gynaecol. 14 (1927) 422-429
Tijssen - in "Practice and theory of enzyme immunoassays" (1990), Amsterdam, Elsevier
Tijssen, P., Hybridization with nucleic acid probes Part I: Theory and nucleic acid preparation in "Laboratory Techniques in Biochemistry and Molecular Biology, Vo" (1993)
Tyagi, S., et al., Proc Natl Acad Sci U S A 93 (1996) 5395-400
Urdea, M. S., Biotechnology (N Y) 12 (1994) 926-8
Vigano, P., et al., Fertil Steril 56 (1991) 894-9
Viville, B., et al., Hum Reprod 12 (1997) 815-22
Walker, G. T., et al., Proc Natl Acad Sci U S A 89 (1992) 392-6
Werth, R. and Grusdew, W., Arch. Gynäkol. 55 (1898) 325-409
Wetzstein, R., Arch. Gynecol. 202 (1965) 1-13
Wu, D. Y. and Wallace, R. B., Genomics 4 (1989) 560-9

US 4,683,195
US 4,683,202
US 5,124,246
US 5,362,655
US 5,510,270
US 5,556,752
US 5,578,832
US 5,629,154
US 5,858,803
WO 01/79846
WO 99/63116

## Claims

1. A method for diagnosing endometriosis, said method comprising
(a) obtaining a sample of menstrual blood
(b) measuring basal cells in said sample or
(c) measuring at least one gene product typical for cells of the basal endometrium in said sample and
(d) correlating the measurement in (b) or (c) to the diagnosis of endometriosis.

2. The method of claim 1, wherein said gene product is a polypeptide.

3. The method of claim 1, wherein basal cells are measured by immunohistology.

4. The method of claim 2, wherein said gene product is selected form the group consisting of estrogen receptor, progesterone receptor and p450 aromatase.

5. The method of claim 2, wherein said polypeptide is measured in an immunoassay.

6. The method of claim 5, wherein said immunoassay is a sandwich immunoassay.

7. The method of claim 1, wherein said gene product is an mRNA.

8. A method for diagnosing endometriosis, said method comprising
(a) obtaining a sample of menstrual blood
(b) measuring the amount of at least one gene product typical for basal cells in said sample,
(c) measuring the amount of a gene product from cells of the functionalis of the endometrium in said sample,
(d) assessing the relative amount of (b) to (c) and
(e) correlating said relative amount (d) to endometriosis.

9. The method of claim 8, wherein said gene product from cells of the functionalis of the endometrium is selected from the group of a protein of the cytoskeleton and 17βHSD-type II.

10. The method of claim 9, wherein said gene product from cells of the functionalis of the endometrium is 17βHSD-type II.
